(19) Europäisches Patentamt
European Patent Office
Office européen des brevets

(11) Publication number: **0 009 655**
**B1**

(12) **EUROPEAN PATENT SPECIFICATION**

(45) Date of publication of patent specification: **11.05.83**

(21) Application number: **79103326.9**

(22) Date of filing: **07.09.79**

(51) Int. Cl.$^3$: **C 07 D 403/12,**
A 61 K 31/50 //(C07D403/12, 207/50, 237/00)

(54) **6-Amino substituted N-pyrrolyl-3-pyridazine amines, their preparation, and pharmaceutically antihypertensive compositions containing them.**

(30) Priority: **02.10.78 GB 3889878**

(43) Date of publication of application:
**16.04.80 Bulletin 80/8**

(45) Publication of the grant of the patent:
**11.05.83 Bulletin 83/19**

(84) Designated Contracting States:
**AT BE CH DE FR GB IT LU NL SE**

(56) References cited:
**AT - B 271 491**
**AU - B - 477 837**
**DE - A - 2 450 873**
**DE - B - 2 308 240**

(73) Proprietor: **GRUPPO LEPETIT S.P.A.**
**8, Via Roberto Lepetit**
**I-20124 Milano (IT)**

(72) Inventor: **Bellasio, Elvio**
**5, Via Volpati**
**Como (IT)**
Inventor: **Di Mola, Nunzio**
**17, Piazza Aspromonte**
**Milano (IT)**
Inventor: **Campi, Ambrogio**
**136, Via Cavallotti**
**Monza (Mi) (IT)**
Inventor: **Baldoli, Emiliana**
**9, Via Besenzanica**
**Milano (IT)**

(74) Representative: **Sgarbi, Renato et al,**
**Patent Department (c/o Gruppo Lepetit S.p.A.)**
**38, Via Durando**
**I-20158 Milano (IT)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

6-Amino substituted N-pyrrolyl-3-pyridazine amines, their preparation, and pharmaceutically antihypertensive compositions containing them

The invention relates to N-pyrrolyl-pyridazineamine derivatives having antihypertensive activity, to the process for their manufacture and to these compounds for use in the treatment of hypertension.

2-(Pyrrol-1-yl)-imidazoleamines having antihypertensive effectiveness are known from U.K. Patent No. 1,408,362. 3-Hydrazinopyridazines and 4-hydrazino-phthalazines having antihypertensive activity are broadly described in the pharmaceutical literature (see Progress in Drug Research, vol. 20, page 203—205, edited by E. Jucker, Birkhäuser Verlag, Basel, 1976).

In Austrian Patent No. 271,491 are described some hydrazones formed by reaction of 3-hydrazino-6-substituted amino-pyridazines with carbonyl compounds, having antihypertensive effect.

The prior literature teaches that blocking of the terminal nitrogen of hydrazine moiety of the 4-hydrazino-phthalazines through chemically stable substituents such as alkyl or aryl groups leads to virtually inactive compounds (see Progress in Drug Research vol. 4 page 322, edited by E. Jucker, Birkhäuser Verlag, Basel, 1962).

The compounds which form the first object of this invention are N-pyrrolyl-pyridazineamine derivatives of the general formula

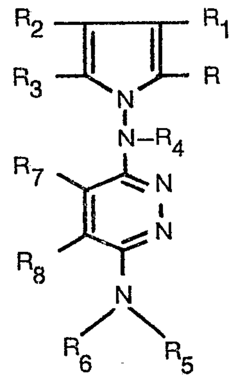

(I)

wherein R, $R_1$, $R_2$, $R_3$, may be the same or different and are independently selected from hydrogen and $(C_1—C_4)$alkyl; $R_4$ represents hydrogen, $(C_1—C_4)$alkyl, $(C_1—C_4)$alkylamino-$(C_1—C_4)$alkyl, di-$(C_1—C_4)$alkylamino$(C_1—C_4)$alkyl, $(C_1—C_4)$-alkanoyl, halo-$(C_2—C_4)$alkanoyl, carbo$(C_1—C_4)$alkoxy or carbobenzyloxy; $R_5$ and $R_6$ each independently represent $(C_1—C_4)$alkyl, hydroxy-$(C_1—C_4)$alkyl, $(C_1—C_4)$alkoxy-$(C_1—C_4)$-alkyl, $(C_1—C_4)$alkanoyloxy-$(C_1—C_4)$alkyl, $(C_3—C_4)$alkenyl, phenyl, phenyl substituted with 1 to 3 substituents independently selected from chloro, fluoro, bromo, $(C_1—C_4)$-alkyl, hydroxy, $(C_1—C_4)$alkoxy, and methylenedioxy, phenyl-$(C_1—C_4)$alkyl and substituted phenyl-$(C_1—C_4)$alkyl wherein the substituents are as above or taken together with the adjacent nitrogen atom represent a saturated 5—6 membered hererocyclic ring which may contain a further heteroatom selected from O, N and S, and which may bear 1 or 2 substituents selected from $(C_1—C_4)$alkyl, phenyl, substituted phenyl wherein the substituents are as above, phenyl-$(C_1—C_4)$alkyl, substituted phenyl-$(C_1—C_4)$alkyl wherein the substituents are as above, hydroxy, hydroxy-$(C_1—C_4)$alkyl and $(C_1—C_4)$alkanoyloxy; $R_7$ and $R_8$ represent hydrogen atoms or, taken together, a 1,3-butadienylene radical forming a benzo system fused with the pyridazine ring; and its pharmaceutically acceptable acid addition salts. In the description of the invention and in the claims the term "$(C_1—C_4)$alkyl" designates a $C_1—C_4$ straight or branched alkyl, preferably methyl and ethyl; the term "$(C_1—C_4)$alkoxy" designates an alkoxy group wherein the aliphatic portion is a straight or branched alkyl of 1 to 4 carbon atoms, preferably a methoxy or an ethoxy group; the term "$(C_1—C_4)$alkanoyl" designates an alkanoyl radical of 1 to 4 carbons, preferably acetyl and propionyl, the term "halo—$(C_2—C_4)$alkanoyl" designates an alkanoyl group of 2 to 4 carbon atoms with 1 to 3 halo substituents, preferably chloroacetyl, fluoroacetyl, trichloroacetyl and trifluoroacetyl; the term "hydroxy-$(C_1—C_4)$alkyl" designates an alkyl group of 1 to 4 carbon atoms with a hydroxy substitution on the chain, preferably, 2-hydroxyethyl, 2-hydroxypropyl and 3-hydroxypropyl; the terms "$(C_1—C_4)$alkylamino-$(C_1—C_4)$alkyl" and "di-$(C_1—C_4)$alkylamino-$(C_1—C_4)$alkyl" designate alkyls of 1 to 4 carbon atoms having one $(C_1—C_4)$alkylamino or di-$(C_1—C_4)$alkylamino substituent such as, for instance, methylamino, dimethylamino, ethylamino and diethylamino; the term "$(C_1—C_4)$alkanoyloxy" designates an alkanoyloxy group of 1 to 4 carbon atoms, preferably formyloxy, acetyloxy and propionyloxy; the term "$(C_1—C_4)$alkoxy-$(C_1—C_4)$alkyl" designates a group wherein the alkoxy portion is defined as before and the alkyl portion is an alkyl of 1 to 4 carbon atoms, preferably ethyl and propyl; the term "$(C_1—C_4)$alkanoyloxy-$(C_1—C_4)$alkyl" designates a group wherein the alkanoyloxy portion is defined as before and the alkyl portion is an alkyl of 1 to 4 carbon atoms, preferably ethyl and propyl; the term

"($C_3$—$C_4$)alkenyl" designates an alkenyl of 3 or 4 carbon atoms, preferably allyl; among the groups defined by the expression "phenyl substituted with 1 to 3 substituents independently selected from chloro, fluoro, bromo, ($C_1$—$C_4$)alkyl, hydroxy ($C_1$—$C_4$)alkoxy and methylenedioxy", preferred are: chlorophenyl, tolyl, methoxyphenyl, dimethoxyphenyl, trimethoxyphenyl, and 3,4-methylenedioxyphenyl; among the groups defined by the expression "substituted phenyl-($C_1$—$C_4$)alkyl" wherein the substituents of the phenyl group are as above, those in which the ($C_1$—$C_4$)alkyl portion is methyl and ethyl are preferred; representative members of the "saturated 5—6 membered heterocyclic rings which may contain a further heteroatom selected from N, O and S", are pyrrolidine, piperidine, piperazine, morpholine and thiomorpholine; typical examples of substitutions on said heterocyclic rings include ($C_1$—$C_4$)alkyl, hydroxy, hydroxy-($C_1$—$C_4$)alkyl and ($C_1$—$C_4$)alkanoyloxy substituents on the carbon atom moiety of said rings and/or ($C_1$—$C_4$)alkyl, hydroxy-($C_1$—$C_4$)alkyl, phenyl, substituted (as above) phenyl, phenyl-($C_1$—$C_4$)alkyl, substituted (as above) phenyl-($C_1$—$C_4$)alkyl substituents on the second nitrogen atoms when the ring contains said further heteroatom.

The phrase "pharmaceutically acceptable acid addition salts" refers to non-toxic acid addition salts of the compounds the anions of which are relatively innocuous to animals at dosages consistent with good antihypertensive activity so that the beneficial pharmacological effect is not vitiated by the side effects ascribable to the anions.

Pharmacologically-acceptable salts include those derived from mineral acids such as hydrochloric acid, hydrobromic acid, sulfuric acid as well as those derived from organic acids such as lactic, maleic, succinic, fumaric, oxalic, glutaric, citric, malic, tartaric, p-toluenesulfonic, benzenesulfonic, methanesulfonic and cyclohexanesulfonic acid. The use of the N-pyrrolyl-pyridazineamines as antihypertensive agents refers to all industrially applicable aspects and acts of said use, including the embodying of the compounds into pharmaceutical compositions. The pharmaceutical compositions containing said active compounds are in fact a further specific object of this invention. The compounds and the compositions of this invention are useful as antihypertensive agents, that is, when said substances are administered in pharmacologically effective amounts to animals suffering of spontaneous or experimentally induced hypertension, produce a considerable reduction of the blood pressure, without displaying any untolerable side effect. The compounds of this invention have the peculiar characteristic of a long lasting action since a remarkable reduction of the blood pressure in the test animals is still persistent even seven hours after administration.

The process for the manufacture of the N-pyrrolyl-pyridazineamines of this invention comprises contacting a hydrazino derivative of the formula

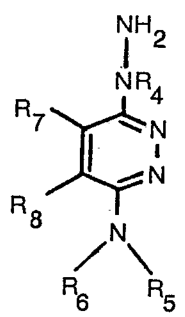

(II)

wherein $R_4$, $R_5$, $R_6$, $R_7$ and $R_8$ have the same meanings as above or an acid addition salt thereof with a dicarbonyl compound of the formula

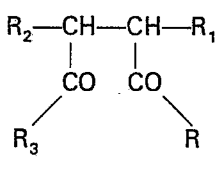

wherein R, $R_1$, $R_2$ and $R_3$ have the same meanings as above or a functional derivative thereof wherein the keto functions can be easily restored under the reaction conditions. The two reactants are usually contacted in about equimolecular amounts although a 1% to 20% excess of dicarbonyl compound may in some instances positively affect the conversion yields of the hydrazine compound.

The reaction is usually carried out in the presence of a solvent such as water, a $C_1$—$C_4$ alkanol, acetic acid, benzene, toluene, tetrahydrofuran, dioxane and mixture thereof, preferably in the presence of an acidic catalyst.

Although several types of catalysts such as hydrohalic acids, sulfuric acid, p-toluenesulfonic acid and Lewis acids may be employed, lower alkanoic acids are particularly suitable in that they may be

used simultaneously both as solvents and catalysts. Among the lower alkanoic acids, acetic acid is particularly preferred. In the case where an acid addition salt of the hydrazine of the formula (II) is employed such as the hydrohalides, the di-hydrohalides, the sulfate and the hydrogen sulfate, the addition of a base or of a basic buffering agent to the reaction solution is needed to allow reaction of the hydrazine in the free base form.

The reaction temperature is generally ranging between 10°C and the boiling temperature of the reaction mixture, preferably between 15°C and 120°C, most preferably between 20°C and 85°C.

The reaction time may vary from 0,5 to 4 hours during which the reaction course may be monitored by thin layer chromatography.

The recovery of the reaction product is carried out according to the general procedures for recovering solid or oily products from organic solutions.

In generic operations, once the reaction is completed, the reaction solution is evaporated to dryness, the residue is slurried with an aqueous solution of a base to remove traces of the acidic catalyst (or solvent); the residue may be dissolved in an organic solvent and then recovered by concentration and/or cooling of the organic solution. The compound thus obtained may be further purified by usual procedures such as crystallization from solvents, column chromatography, preparative thin layer chromatography and similar methods.

The acid addition salts of the compounds of formula (I) may be obtained through common procedures from the corresponding free bases by addition of an appropriate acid. The intermediate hydrazines of formula (II) are prepared according to procedures described in the literature. Some hydrazino-pyridazines are specifically described for instance in the following U.K. patents: U.K. 1.157.642, U.K. 1.373.548, U.K. 1.299.421 and in the following papers: E. Bellasio et al. Il Farmaco Ed. Sci. *24*, 921 (1969); G. Pifferi et al. J. Med. Chem. *18*, 741 (1975).

For preparation of compounds of formala (I) wherein $R_4$ represents $(C_1—C_4)$alkyl, $(C_1—C_4)$alkanoyl, carbo-$(C_1—C_4)$-alkoxy, or carbobenzyloxy, besides the general method described above, a further procedure may be advantageously employed in cases where the preparation of the corresponding N-alkylated or acylated hydrazine starting material is difficult. This alternative procedure involves preparation of the compounds of formula I wherein $R_4$ is hydrogen which are then alkylated or acylated on the unsubstituted nitrogen-atom by means of common alkylation or acylation procedures.

Alkylation may be carried out, for instance, with $(C_1—C_4)$-alkyl halides or sulfates in the presence of acid acceptors such as alkali metal hydrides and alkali metal alkoxides. Acylation may be carried out by reacting the N-unsubstituted compound with the appropriate acyl halide or anhydride, optionally in the presence of an acid acceptor such as pyridine.

The acylation procedure may also be employed for conversion of free hydroxy groups to $(C_1—C_4)$ alcyloxy in the portion

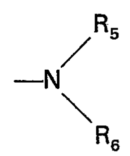

of the compounds of formula (I) above. Representative examples of compounds of this invention are indicated in the TABLE.

The antihypertensive activity of the compounds of the invention was shown in representative tests on spontaneous hypertensive rats and in renal hypertensive dogs. In representative experiments which renal hypertensive dogs, effective amounts (1 to 4 mg/kg) of compounds of examples 3, 4, 5, 6, 8, 13, 14 and 15 were administered p.o. to the conscious hypertensive animals. The systolic arterial blood pressure was measured by the indirect method on the tail before and 1, 3, 5 and 7 hours after treatment.

The results of these experiments showed that the compounds were effective in lowering the blood pressure. The drop of the systolic blood pressure ranged between 20 and 70 mm Hg, depending on the specific compound tested and on the time at which the blood pressure was observed. In general the antihypertensive effect started about 3 hours after treatment and the maximum effect was still persistent 7 hours after treatment. In representative experiments with spontaneous hypertensive rats (MHR: Milano hypertensive rats; see G. Bianchi et al.—Clinical and Experimental Pharmacology and Physiology, Suppl. 3, 15—20, 1976) a 20 to 60 mm Hg systolic blood pressure drop was observed in the test animals administered p.o. with effective (2 to 100 mg/kg) amounts of the compounds of Examples 1, 2, 3, 4, 5, 6, 7, 8, 9, 11, 12, 13, 14, 15, 16, 17, 18, 19, 20, 21, 22, 23, 25 and 26.

The toxicity of these compounds was found to be very low since the values of the $LD_{50}$ in mice were generally higher than 500 mg/kg p.o. and, in most cases, the $LD_{50}$ was greater than 800 mg/kg p.o. Besides, representative compounds of this invention have shown absence of any mutagenic effect in current mutagenetic *in vitro* tests (See Ames et al.: Proc. Natl. Acad. Sci. U.S., 70:782, 1973).

For instance, the compound of example 3 having a value of $LD_{50}$ of about 800 mg/kg p.o. provoked a blood pressure drop of 38 mm Hg, when administered at a dose of 5 mg/kg to spontaneous

hypertensive rats. In renal hypertensive dogs, drops of the systolyc blood pressure of 50 and 70 mm Hg, were observed with doses of 1 and 4 mg/kg p.o. respectively. The hydrochloride of said compound of example 3 as well as the compounds of examples 2, 19 and 21 showed analogous results.

A surprising and very interesting pharmacological effect showed by these compounds is the persistence of the antihypertensive action even at a considerable period of time after the administration. For instance, the compound of example 3, when administered at a dose of 2 mg/kg to renal hypertensive dogs, showed a maximum systolic blood pressure drop of 52 mm Hg after about three hours and after seven hours a blood pressure drop of 48 mm Hg was still registered. Under the same conditions and at the same dosage, hydralazine (1-hydrazinophthalazine) showed a maximum decrease of the systolic blood pressure of 41 mm Hg after about one hour but, after seven hours, the blood pressure decrease was only of 29 mm Hg.

The effect was confirmed in experiments wherein the two substances were administered intravenously at equipotent dosages.

Another very remarkable effect of the antihypertensive compounds of this invention is that the maximum blood pressure drop is reached through a gradual decrease which does not dramatically affect all the circulatory parameters concerned, thus avoiding the undesired side-effects generally displayed by most of the known antihypertensive substances.

The persistence of the antihypertensive effect is a very favorable characteristic of the compounds of this invention, since it allows a less frequent administration schedule and, moreover, the total amount of antihypertensive substance required to keep the blood pressure value at a normal level in chronically hypertensive patients is lower than with other hypertensive substances having the same potency but with a shorter period of action.

The following are examples of preparation of representative compounds of this invention given by way of illustration only, without any intention of limiting the present invention.

## Example 1

N-(2,5-Diethyl-1H-pyrrol-1-yl)-6-morpholino-3-pyridazineamine

To 1.95 g (10 m moles of 3-hydrazino-6-morpholino-pyridazine dissolved in 12 ml of acetic acid, 1.71 g (12 m moles) of 3,6-octanedione dissolved in 4 ml of acetic acid are added at room temperature. The mixture is heated at 67°C for 2 hours and then evaporated to dryness under vacuum. The oily residue is dissolved in toluene and the solution is evaporated to dryness. The residue is slurried with ice-water and neutralized with sodium bicarbonate to yield 2.58 g of a solid product. Said material, after crystallization from isopropanol gives 1.25 (42%) of the title product, melting at 186—189°C.

Elemental analysis, I. R., N.M.R. and mass spectra are in agreement with the assigned structure.

## Example 2

6-Diethylamino-N-(2,5-dimethyl-1H-pyrrol-1-yl)-3-pyridazineamine

To 105 ml of acetic acid, 15.3 g (60 m moles) of 6-diethylamino-3-hydrazino-pyridine dihydrochloride, 9.85 g (120 m moles) of sodium acetate and 7.51 g (66 m moles) of 2,5-hexanedione are added and the mixture is heated at 67°C for 3.5 hours.

The solvent is evaporated off under vacuum yielding an oily residue which is dissolved in toluene. The toluene solution is evaporated and the residue obtained, is slurried with ice-water and neutralized with a saturated sodium carbonate solution. After extraction of the mixture with three portions of 150 ml of chloroform, the organic layer is washed with water and evaporated in vacuo to yield 17 g of crude product. Said material is purified by chromatography through a silicagel column by eluting with cyclohexane-ethyl acetate mixtures wherein the ratio of ethyl acetate to cyclohexane is gradually increased from 1:4 to 1:1.

By evaporation of the eluate 6 g (39%) of the product of the title are obtained.

After crystallization from isopropanol the compound melts at 148—150°C.

Elemental analysis, I.R. and N.M.R. data are in agreement with the assigned structure.

## Example 3

N-(2,5-Dimethyl-1H-pyrrol-1-yl)-6-morpholino-3-pyridazineamine

a) 8.25 Grams (42.3 m moles) of 3-hydrazino-6-morpholino pyridazine and 5.82 g (51 m moles) of 2,5-hexanedione in 40 ml of acetic acid are heated at 67°C for 3.25 hours. The solvent is evaporated off under vacuum and the residue is slurried with ice-water and neutralized with aqueous sodium carbonate.

The product is purified through column chromatography on silicagel by eluting with cyclohexane-ethyl acetate mixtures wherein the ratio of ethyl acetate to cyclohexane is gradually increased from 1:4 to 2:1. The fractions containing the purified product are combined and evaporated to dryness giving a solid residue which, after crystallization from isopropanol, melts at 191—193°C (yield 6 g, 52%).

Elemental analysis, I.R. and N.M.R. data are in agreement with the assigned structure.

b) The same compound is obtained also by following the procedure of example 2 using 14 g of 3-hydrazino-6-morpholino-pyridiazine dihydrochloride, 8.6 g of sodium acetate and 6.52 g of 2,5-hexanedione in 90 ml of acetic acid. The yield is 9.5 g (48%) of the product of the above title.

c) A further procedure to prepare the compound of the title is the following:

To 58.6 g (200 m mol) of 6-hydrazino-3-(4-morpholino)-pyridazine sulfate dissolved in 100 ml of water, 200 ml of 1M sodium hydroxide are gradually added under cooling and when this addition is completed, 25 g (220 m mol) of 2,5-hexanedione are added to the mixture which then is heated at 70°C for four hours. After cooling to about 20°C, a further addition of 200 ml of 1N sodium hydroxide is made and the product precipitated is removed by filtration and washed on filter with three portions of 100 ml of ice-water. After drying at 50°C over $P_2O_5$ the solid weights 54.4 g (99%). The product shows satisfactory analytical characteristics. The 6-hydrazino-3-(4-morpholino)-pyridazine sulfate is obtained by adding the stoichiometric amount of $H_2SO_4$ to an aqueous solution of the hydrazine and evaporating off the water. The sulfate melts at 202—4°C when crystallized from ethanol containing 10% of water.

The hydrochloride of the compound of the title is obtained by dissolving 17 g of the free base in 150 ml of absolute ethanol at 70°C and then adding to said solution 45 ml of ethyl ether saturated with hydrogen chloride. Further addition of 300 ml of ethyl ether to the cooled solution yields a precipitate which after filtation is crystallized from 200 ml of 85% ethanol. Yield 13.5 g; the compound decomposes at 260°C.

Example 4

6-(2,6-Dimethyl-morpholino)-N-(2,5-dimethyl-1H-pyrrol-1-yl)-3-pyridazineamine

The compound is prepared according to the procedure of example 2 by contacting 3.8 g (12.8 m moles) of 6-(2,6-dimethyl-morpholino)-3-hydrazino-pyridazine dihydrochloride (prepared according to the procedure described in U.K. patent 1.157.642; m.p. 217—220°C), 2.2 g (25.6 m moles) of sodium acetate and 1.61 g (14.1 m moles) of 2,5-hexanedione in 20 ml of acetic acid. The yield is 1.8 g (47%) of the product of the title which melts at 147—148°C when crystallized from ethyl ether.

Elemental analysis, I.R. and N.M.R. data are in agreement with the assigned structure.

Example 5

N-(2,5-Dimethyl-1H-pyrrol-1-yl)-4-mopholino-1-phthalazineamine

To 4.9 grams (20 m moles) of 1-hydrazino-4-morpholinophthalazine (prepared from 1,4-dichlorophthalazine according to the procedure in U.K. patent 1.157.642; M.p. 255—260°C) dissolved in 30 ml of acetic acid, 2.74 g (24 m moles) of 2,5-hexanedione are added and the mixture is heated at 65°C for 3 hours.

The solvent is evaporated off under vacuum and the residue is slurried with water and neutralized with a saturated solution of sodium carbonate. The raw product is extracted with ethyl acetate and the organic solution, after washing with water and drying over $CaSO_4$ is evaporated to dryness. The residual product is purified by chromatography through a silicagel column using cyclohexane-ethyl acetate 1:3 as the eluent. Evaporation of the eluate, yields 2.7 g (47%) of the product of the title which after crystallization from acetone, melts at 205—209°C.

Elemental analysis, I.R. and N.M.R. data are in agreement with the assigned structure.

Example 6

6-Diallylamino-N-(2,5-dimethyl-1H-pyrrol-1-yl)-3-pyridazineamine

To 7 g (25 m moles) of 6-diallylamino-3-hydrazino-pyridazine dihydrochloride (Ger. Appl. 2.002.107; C.A. *73*, 66596, 1970), and 4.1 g (50 m moles) of sodium acetate dissolved in 40 ml of acetic acid, 3.42 g (30 m moles) of 2,5-hexanedione are added.

After heating for 4 hours at 65°C, the mixture is evaporated to dryness. The residue is slurried with ice-water and neutralized with sodium carbonate. The mixture is extracted with ethyl acetate and the raw product obtained by evaporation of the organic extract (7 g) is purified by chromatography through a silicagel column using chloroform and a mixture chloroform-methanol 98.5:1.5 as the eluent. Evaporation of the eluate yields a product which, after crystallization from ethyl ether, melts at 135—136°C (2.6 g, 37%).

Elemental analysis, I.R. and N.M.R. data are in agreement with the assigned structure.

Example 7

N-(2,5-Dimethyl-1H-pyrrol-1-yl)-6-(1-pyrrolidinyl)-3-pyridazineamine

To a mixture of 5.2 g (20.7 m moles) of 3-hydrazino-6-(1-pyrrolidinyl)pyradazine dihydrochloride (prepared according to U.K. patent 1.157.642. The compound was characterized through the dihydrochloride of the corresponding hydrazone with acetone melting at 215—220°C) and 3.56 g (43 m moles) of sodium acetate in 60 ml of acetic acid, 2.74 g (24 m moles) of 2,5-hexanedione are added. After stirring for 3 hours at 65—55°C the solvent is evaporated off under vacuum.

The residue is slurried with water, neutralized with sodium bicarbonate and then dissolved in chloroform. The chloroform solution is chromatographed through silicagel by eluting with cyclohexane-ethyl acetate mixtures wherein the ratio of ethyl acetate to cyclohexane is gradually increased from 1:1 to 4:1. The eluate is evaporated to dryness and the solid residue is crystallized from ethyl acetate yielding 3.35 g (63%) of the product of the title which melts at 208—209°C.

Elemental analysis, I.R. and N.M.R. data are in agreement with the assigned structure.

Example 8

N-(2,5-Dimethyl-1H-pyrrol-1-yl)-6-piperidino-3-pyridazineamine

A mixture of 7.4 g (28 m moles) of 3-hydrazino-6-piperidino-pyridazine, 2.3 g of sodium acetate and 3.7 g 2,5-hexanedione is heated for two hours at 65°C. The solvent is evaporated off under vacuum and the solid residue is dissolved in water and neutralized with sodium hydroxide. The product is purified by chromatography through a silicagel column using as the eluent a mixture methanol-chloroform 2.5:97.5. The solid recovered by evaporation of the eluate is crystallized from ethyl acetate yielding 2 g (27%) of the product of the title which melts at 185—187°C.

Elemental analysis; I.R. and N.M.R. data are in agreement with the assigned structure.

Example 9

N-(2,5-Dimethyl-1H-pyrrol-1-yl)-6-(4-methyl-1-piperazinyl)-3-pyridazineamine

A mixture of 7 g (35 m moles) of 3-hydrazino-6-(4-methyl-1-piperazinyl)-pyridazine and 4.1 g (36 m moles) of 2,5-hexanedione in 100 ml of acetic acid is heated for 3 hours at 70°C. The solvent is evaporated off under vacuum and the oily residue is dissolved in water and made alkaline with 10% sodium hydroxide. The mixture is extracted with ethyl acetate and the organic layer is evaporated to dryness in vacuo. The solid residue is crystallized first from ethyl ether and then from ethyl acetate yielding 2.6 g (26%) of the product of the title which melts at 181—182°C.

Elemental analysis, I.R. and N.M.R. data are in agreement with the assigned structure.

Example 10

N-(2,5-Dimethyl-1H-pyrrol-1-yl)-6-(1-piperazinyl)-3-pyridazineamine

A mixture of 9.2 g (40 m moles) of 3-hydrazino-6-(1-piperazinyl)-pyridazine hydrochloride (m.p. 257°C; prepared according to the procedure of U.K. patent 1.157.642), 3.28 g (40 m moles) of ethyl acetate and 4.82 g of 2,5-hexanedione in 100 ml of acetic acid is heated at 75°C for 2 hours and then allowed to stand for two days at room temperature.

After filtration, the solvent is evaporated off in vacuo and the residue is dissolved in water and made alkaline with 10% sodium hydroxide. Extraction with ethyl acetate and evaporation of the organic layer, yields 4.1 g of crude product which is purified by chromatography through a silicagel column using a methanol-chloroform 7:3 as the eluent. Yield 3 g (28%) of the title product which after crystallization from acetonitrile melts at 189—191°C.

Elemental analysis, I.R. and N.M.R. data are in agreement with the assigned structure.

Examples 11—15

The following products are prepared by contacting the corresponding hydrazinopyridazine dihydrochlororides and 2,5-hexanedione in the presence of ethyl acetate according to the procedure described in example 2.

11) N-(2,6-Dimethyl-1H-pyrrol-1-yl)-6-[N',N'-bis(2-hydroxyethyl)amino]-3-pyridazineamine. M.p. 128—130°C. Yield 65%.

12) N-(2,6-Dimethyl-1H-pyrrol-1-yl)-6-[N'-(2-hydroxyethyl)-N'-(2-hydroxypropyl)amino]-3-pyridazineamine. M.p. 129—131°C. Yield 40%.

13) N-(2,6-Dimethyl-1H-pyrrol-1-yl)-6-[N'-(2-hydroxyethyl)-N'-methyl-amino]-3-pyridazineamine. M.p. 139—140°C. Yield 64%.

14) N-(2,6-Dimethyl-1H-pyrrol-1-yl)-6-[N',N'-bis(2-hydroxypropyl)amino]-3-pyridazineamine. M.p. 137—139°C. Yield 45%.

15) N-(2,6-Dimethyl-1H-pyrrol-1-yl)-6-(4-hydroxy-piperidino)-3-pyridazineamine. M.p. 175—177°C. Yield 30%.

The starting hydrazino-pyridazine compounds of examples 11 to 15 are literature compounds. The starting material of example 15 is prepared according to the procedure described in U.K. patent 1.157.642 and is employed as such for the further reaction without isolation and characterization.

Example 16

N-(2,5-Dimethyl-1H-pyrrol-1-yl)-N-methyl-6-morpholino-3-pyridazineamine

To 1.36 g (5 m moles) of N-(2,5-dimethyl-1H-pyrrol-1-yl)-6-morpholino-3-pyridazineamine in 13.6 ml of dimethylformamide, 0.26 g (5.5 m moles) of 55% sodium hydride are added. The mixture is stirred for 30 minutes at room temperature and for additional 30 minutes at 55°C. Then, a solution of 0.78 g (5.5 m moles) of methyl iodide in 2 ml of dimethylformamide is gradually added at 10°C. When the addition is completed the mixture is heated at 50°C; for 45 minutes.

The dimethylformamide is evaporated off in vacuo and the residue is dissolved in ethyl acetate. The organic layer is washed with water and then evaporated to dryness to give a solid which is crystallized from hexane. Yield 0.7 g (54%) of the product of the title which melts at 119—122°C.

Elemental analysis, I.R. and N.M.R. data are in agreement with the assigned structure.

Example 17

N-Acetyl-N-(2,5-dimethyl-1H-pyrrol-yl)-6-morpholino-3-pyridazineamine

A mixture of 6 g (22 m mol) of N-(2,5-dimethyl-1H-pyrrol-yl)-6-morpholino-3-pyridazineamine, 30 ml of acetic anhydride, and 6 ml of pyridine is heated for one hour at 110°C. The reaction mixture is evaporated to dryness under vacuum and the oily residue is dissolved in 150 ml of ethyl acetate. The organic solution is washed first with 50 ml of an aqueous solution of sodium bicarbonate and then with 50 ml of water.

The organic layer is dried over $CaSO_4$ and evaporated to yield an oily residue which is chromatographed on a silicagel colum using cyclohexane-ethyl acetate 3:2 as the eluent.

After evaporation of the eluate the oily residue dissolved in ethyl ether is additioned with a hydrogen chloride ethyl ether solution.

The solid precipitate, which is highly hygroscopic, is recovered by filtration and crystallized from isopropanol-ethyl ether 1:1. Yield 4.9 g (64%) of the product of the title (hydrochloride) melting at 162—168°C.

Elemental analysis, I.R. and N.M.R. data are in agreement with the assigned structure.

Example 18

N-(2,5-Dimethyl-1H-pyrrol-1-yl)-6-(4-thiomorpholinyl)-3-pyridazineamine

3-Hydrazino-6-(4-thiomorpholinyl)-pyridazine di-hydrochloride and 2,5 hexanedione are reacted in acetic acid in the presence of sodium acetate according to the procedure of example 2. The product is recovered by evaporating the acetic acid and slurring the residue in aqueous sodium bicarbonate. The solid obtained after filtration is purified by chromatography through silicagel using a chloroform-methanol 97.5:2.5 mixture as the eluent. Yield 50%. M.p. 203°C (from ethyl acetate).

Elemental analysis, I.R. and N.M.R. data are in agreement with the assigned structure.

Example 19

N-(2,5-Dimethyl-1H-pyrrol-1-yl)-6-[N',N'-bis(2-methoxyethyl)amino]-3-pyridazineamine

The compound is obtained according to the same procedure described in example 2 by reacting 3-hydrazino-6-[N,N-bis(2-methoxyethyl)amino]-pyridazine dihydrochloride and 2,5-hexanedione in acetic acid in the presence of sodium acetate. The chromatographic purification on a silicagel column is carried out by using a mixture ethyl acetate-cyclohexane 3:1 as the eluent. Yield 60% M.p. 112—114°C (from ethyl acetate).

The 3-hydrazino-6-[N,N-bis(2-methoxyethyl)amino]-pyridazine dihydrochloride, m.p. 198—200°C, is obtained by employing the procedures of methods E, F, G described by G. Pifferi et al. in J. Med. Chem., *18*, 741 (1975).

Example 20

N-(2,5-Dimethyl-1H-pyrrol-1-yl)-6-[N'-methyl-N'-(2-methoxyethyl)amino]-3-pyridazineamine

The compound is prepared according to the procedure of example 19 from 3-hydrazino-6-[N-methyl-N-(2-methoxyethyl)amino]-pyridazine dihydrochloride and 2,5-hexanedione in acetic acid in the presence of sodium acetate. Yield 55%. M.p. 106°C (from ethyl ether). The dihydrochloride of 3-hydrazino-6-[N-methyl-N-(2-methoxyethyl)amino]-pyridazine, m.p. 219—221°C, is obtained by employing the procedures of methods E, F, G described by G. Pifferi et al. in J. Med. Chem., *18*, 741 (1975).

Example 21

N-(2,5-Dimethyl-1H-pyrrol-1-yl)-6-[N',N'-bis(2-ethoxyethyl)amino]-3-pyridazineamine

The compound is prepared according to the procedure of example 19, from 3-hydrazino-6-[N,N-bis(ethoxyethyl)amino]-pyridazine dihydrochloride and 2,5-hexanedione in acetic acid, in the presence of sodium acetate. Yield 70%. B.p. 180°C/0.2 mm Hg. The dihydrochloride of 3-hydrazino-6-[N,N-bis(ethoxyethyl)amino]-pyridazine, m.p. 181—183°C, is obtained by employing the procedures of methods E, F, G described by G. Pifferi et al. in J. Med. Chem., *18*, 741 (1975).

Example 22

N-(2,5-Dimethyl-1H-pyrrol-1-yl)-6-dimethylamino-3-pyridazineamine

The product is obtained according to the procedure of example 19 by reacting 3-hydrazino-6-dimethylamino-pyridazine dihydrochloride and 2,5-hexanedione in acetic acid in the presence of sodium acetate. Yield 47%; m.p. 165—167°C (from ethyl ether).

Example 23

N-(2,5-Dimethyl-1H-pyrrol-1-yl)-6-[4-(2-methoxyphenyl-1-piperazinyl]-3-pyridazineamine

The product is obtained according to the procedure of example 19 by reacting 3-hydrazino-6-[4-(2-methoxyphenyl-1-piperazinyl]-pyridazine and 2,5-hexanedione in acetic acid in the presence of sodium acetate. Yield 51%, m.p. 194—196°C. The starting hydrazine is prepared from 3,6-dichloropyridazine according to the process described in U.K. Patent 1.157.642. The 3-chloro-6-[4-(2-methoxyphenyl)-1-piperazinyl]-pyridazine intermediate melts at 141—143°C. The hydrazine is not

characterized as a free base but is employed as such for the reaction with 2,5-hexanedione. The benzilidene hydrazone of said hydrazine melts at 230—233°C (from methanol).

Example 24

6-Morpholino-N-(1H-pyrrol-1-yl)-3-pyridazineamine

To 5.85 g (30 m mol) of 3-hydrazino-6-morpholino-pyridazine in 130 ml of ethanol, ethyl ether (45 ml) saturated with hydrogen chloride is added. The suspension of pale yellow precipitate which forms is additioned with 12 g (90 m mol) of 2,5-dimethoxytetrahydrofuran and the mixture is refluxed (60°C) for 6 hours. The solvent is removed under vacuum and the residue is dissolved in water, brought to pH 8 by addition of a sodium carbonate solution and extracted with four portions (each of 100 ml) of dichloromethane. The organic extracts are pooled together, washed with water (50 ml) and anhydrified over sodium sulfate. Evaporation of the solvent yields an oil which is purified through column chromatography (silicagel, 500 g) using as the eluent a mixture of dichloromethane and ethyl acetate wherein the ratio of ethyl acetate is gradually increased from 20% to 100%. Evaporation of the more polar fraction yields 0.15 g (2%) of the product of the title which melts at 228°C.

Elemental analysis, I.R. and N.M.R. data confirm the assigned structure.

Example 25

N-Methyl-6-morpholino-N-(1H-pyrrol-1-yl)-3-pyridazineamine

To a solution of 6.76 g (22.5 m mol) of 3-(1-methylhydrazino)-6-morpholino-pyridazine dihydrochloride monohydrate in 135 ml of ethanol, a saturated solution of hydrogen chloride in ethyl ether (18 ml) and 4.46 g (33.75 m mol) of 2,5-dimethoxytetrahydrofuran are added. After refluxing for 3 hours, the solvent is evaporated off under vacuum and the residue, dissolved in water is neutralized with aqueous sodium bicarbonate. After extraction with three portions (each of 200 ml) of dichloromethane, the extracts are combined and anhydrified over sodium sulfate. Evaporation of the solvent yields a product which is purified through a silicagel column (350 g) using a mixture ethyl acetate-cyclohexane 1:3 as the eluent. Crystallization from ethyl ether yields 2.31 g (50%) of the product of the title, which melts between 105° and 117°C. (Thermal analysis shows that two crystalline forms are present, one melting at 105°C and the other at 117°C).

Elemental analysis, I.R. and N.M.R. data confirm the assigned structure.

The 3-(1-methylhydrazino)-6-morpholino-pyridazine dihydrochloride monohydrate is prepared through the following procedure:

The hydrazone of 3-hydrazino-6-morpholino-pyridazine with acetaldehyde is prepared from 3-hydrazino-6-morpholino-pyridazine and acetaldehyde in water solution; m.p. 175—180°C. The hydrazone, after drying, is heated (55°C, 30 minutes) with a slight excess of 55% sodium hydride in dimethylformamide. To the suspension is then added a slight excess of methyl iodide and heated at 53°C for 90 minutes. Evaporation of the solvent yields a residue which is dissolved in ethyl acetate, washed with water and dried over sodium sulfate. Removal of ethyl acetate and taking up the residue with cyclohexane yields a product which is used for the further step. A sample of the acetaldehyde methyl (6-morpholino-3-pyridazinyl)hydrazone crystallized from ethyl ether melts at 136°C.

The acetaldehyde hydrazone is hydrolyzed according to the procedure of method G described by G. Pifferi et al. in J. Med. Chem., *18*, 741 (1975). The 3-(1-methylhydrazino)-6-morpholino-pyridazine dihydrochloride monohydrate, crystallized from methanol, melts at 183—190°C (softening at 177°C).

Example 26

N-(2,5-Dimethyl-1H-pyrrol-1-yl)-4-(4-hydroxypiperidino)-1-phthalazineamine

To a solution of 1.5 g (5.8 m mol) of 1-hydrazino-4-(4-hydroxypiperidino)-phthalazine in 60 ml of acetic acid, 0.79 g (6.9 m mol) of 2,5-hexanedione are added and the mixture is heated at 67°C for 3 hours. After evaporation of the solvent the residue is neutralized with aqueous sodium bicarbonate and extracted with ethyl acetate. The organic solution is chromatographed through a silicagel column using ethyl acetate as the eluent. Yield 0.5 g (26%) of the product of the title which melts at 180—185°C.

Elemental analysis, I.R. and N.M.R. data confirm the assigned structure.

The 1-hydrazino-4-(4-hydroxypiperidino)-phthalazine is prepared by reacting 1,4-dichlorophthalazine with 4-hydroxypiperidine to yield 1-chloro-4-(4-hydroxypiperidino)-phthalazine (m.p. 139—142°C) and then converting this latter to the corresponding hydrazino derivative by reaction with an excess of hydrazine hydrate. These reactions are carried out according to the procedure described in U.K. Patent 1.157.642 for analogous hydrazine pyridazine derivatives.

The 1-hydrazino-4-(4-hydroxypiperidino)-phthalazine melts at 190—192°C (from isopropanol).

In the exploitation of the invention, the preferred administration route of the compounds of this invention is per os in the form of capsules, tablets, troches, lozanges, granules, suspensions, syrups, elixirs or solutions. If desired, for severe cases, parenterally administrable dosage forms can also be prepared as injectable ampoules. The dosage forms for oral use are prepared by common procedures. Capsules, besides the active ingredient may contain pharmaceutically acceptable excipients, such as, for instance, dextrin, starch, lactose, cellulose derivatives, and magnesium stearate. Coated or hard shell capsules can also be prepared. Tablets may include inert diluents such as lactose, glucose and

talc, granulating and disintegrating agents such as starch and alginic acid; binding agents; and lubricating agents such as magnesium stearate, talc etc. For example, a gelatin capsule suitable as a dose unit may contain 10 mg of the compound of example 3 or its hydrochloride, 1.5 mg of magnesium stearate and 118.5 mg of corn starch. Other possible oral dosage forms such as suspensions, syrups and elixirs are formulated as known in the art (see for instance the book "Remington's Pharmaceutical Sciences", 13th Ed. Mack Publishing Co. Easton, Pennsylvania) and may contain suspending agents, such as methyl cellulose, tragacanth or alginates; wetting agents such as polyoxyethylene sorbitan monoleate; and preservatives. The liquid solutions for both oral and parenteral use may contain antioxidants, preservatives, buffering agents, and dispersing or wetting agents. The solvents which may be employed generally are water or mixture of water and polihydric aliphatic alcohols. For instance, a suitable dose unit form for extemporaneous parenteral use may be prepared by dissolving the content of a lyophilized vial consisting of 3 mg of the compound of example 3 or its hydrochloride, 50 mg of mannitol and 0.5 mg of disodium edetate in 10 ml of water for injection.

In general, the antihypertensive effective amount of the compounds of this invention depend on several factors such as the particular compound administered, the body weight, the severity and the origin of the hypertensive disorders, the effects and the nature of other pharmacologically active substances which may be associated thereto in the treatment of the hypertension. In general, the treatment of hypertensive disorders with the pyrrolyl-pyridazineamines of this invention may be started with low dosages which may be generally increased according to the individual response. The antihypertensive effective dosage in oral administration usually ranges from about 0.10 mg/kg to about 3 mg/kg, with the daily dosage of from about 0.25 mg/kg to about 2 mg/kg being preferred. In parenteral administration, the antihypertensive effective dosage generally ranges from about 0.01 mg/kg to about 1.5 mg/kg daily, the dosage range from about 0.03 mg/kg to about 1 mg/kg daily being preferred.

It is however clear that a dose beyond the above indicated ranges may also be employed depending on the individual conditions of the subject to be treated.

TABLE

| R | $R_1$ | $R_2$ | $R_3$ | $R_4$ | $R_5$ | $R_6$ | $R_7$ | $R_8$ |
|---|---|---|---|---|---|---|---|---|
| $CH_3$ | H | H | $CH_3$ | H | $-C_2H_5$ | $-C_2H_5$ | H | H |
| $CH_3$ | H | H | $CH_3$ | H | $-CH_2-CH_2-CHOH-CH_2-CH_2-$ | | H | H |
| $CH_3$ | H | H | $CH_3$ | H | $-CH_2-CH_2-NH-CH_2-CH_2-$ | | H | H |
| $CH_3$ | H | H | $CH_3$ | H | $-CH_2-CH_2-N(CH_3)-CH_2-CH_2-$ | | H | H |
| $CH_3$ | H | H | $CH_3$ | H | $-CH_3$ | $-CH_2-CHOH-CH_3$ | H | H |
| $CH_3$ | H | H | $CH_3$ | H | $-CH_2-CH_2OH$ | $-CH_2-CH_2OH$ | H | H |
| $CH_3$ | H | H | $CH_3$ | H | $-CH_2-CH_2OH$ | $-CH_2-CHOH-CH_3$ | H | H |
| $CH_3$ | H | H | $-CH_3$ | H | $-CH_2-CH=CH_2$ | $-CH_2-CH=CH_2$ | H | H |
| $CH_3$ | H | H | $-CH_3$ | $-CH_2-CH_2-N(CH_3)_2$ | $-CH_2-CH_2-O-CH_2-CH_2-$ | | H | H |
| $CH_3$ | H | H | $CH_3$ | H | $-CH_2-CH_2-CH_2-CH_2-$ | | H | H |
| $CH_3$ | H | H | $CH_3$ | H | $-CH_2-CH_2-CH_2-CH_2-CH_2-$ | | H | H |
| $CH_3$ | H | H | $CH_3$ | H | $-CH_2-CH_2-O-CH_2-CH_2-$ | | H | H |
| $CH_3$ | H | H | $CH_3$ | $CH_3$ | $-CH_2-CH_2-O-CH_2-CH_2-$ | | H | H |
| $CH_3$ | H | H | $CH_3$ | H | $-CH_2-CH(CH_3)-O-CH(CH_3)-CH_2$ | | H | H |
| $CH_3$ | H | H | $CH_3$ | H | $-CH_2-CHOH-CH_3$ | $-CH_2-CHOH-CH_3$ | H | H |
| $CH_2H_5$ | H | H | $C_2H_5$ | H | $-CH_2-CH_2-O-CH_2-CH_2-$ | | H | H |
| $CH_3$ | H | H | $CH_3$ | $-COCH_3$ | $-CH_2-CH_2-O-CH_2-CH_2-$ | | H | H |

| R | $R_1$ | $R_2$ | $R_3$ | $R_4$ | $R_5$ | $R_6$ | $R_7$ | $R_8$ |
|---|---|---|---|---|---|---|---|---|
| $CH_3$ | H | H | $CH_3$ | H | $-CH_2-CH_2-O-CH_2-CH_2-$ | | $-CH=CH_2-CH=CH_2-$ | |
| $CH_3$ | H | H | $CH_3$ | H | $-CH_2-CH_2-OCH_3$ | $-CH_2-CH_2OCH_3$ | H | H |
| $CH_3$ | H | H | $CH_3$ | H | $-CH_2-CH_2-OC_2H_5$ | $-CH_2-CH_2-OC_2H_5$ | H | H |
| $CH_3$ | H | H | $CH_3$ | H | $-CH_3$ | $-CH_2-CH_2OCH_3$ | H | H |
| H | H | H | H | H | $-CH_2-CH_2-O-CH_2-CH_2-$ | | H | H |
| $CH_3$ | $CH_3$ | $CH_3$ | $CH_3$ | H | $-CH_2-CH_2-OCH_2-CH_2-$ | | H | H |
| $CH_3$ | H | $CH_3$ | H | H | $-CH_2-CH_2-O-CH_2-CH_2-$ | | H | H |
| $CH_3$ | H | H | $CH_3$ | H | $-CH_2-CH_2-S-CH_2-CH_2-$ | | H | H |
| $CH_3$ | H | H | $CH_3$ | H | $-CH_2-CH_2OCOCH_3$ | $-CH_2-CH_2OCOCH_3$ | H | H |
| $CH_3$ | H | H | $CH_3$ | H | $-CH_3$ | $-C_6H_5$ | H | H |
| $C_2H_5$ | $CH_3$ | $CH_3$ | $C_2H_5$ | H | $-CH_2-CHOH-CH_3$ | $-CH_2-CHOH-CH_3$ | H | H |
| $CH_3$ | H | H | $CH_3$ | H | $-CH_2-CH_2-N[(2\text{-}OCH_3)C_6H_4]-CH_2-CH_2-$ | | H | H |
| $CH_3$ | H | H | $CH_3$ | H | $-CH_2-CH_2-N[(4\text{-}Cl)C_6H_4]-CH_2-CH_2-$ | | H | H |
| $CH_3$ | H | H | $CH_3$ | H | $-CH_2-CH_2-N[(3\text{-}CH_3)C_6H_4]-CH_2-CH_2-$ | | H | H |
| $CH_3$ | H | H | $CH_3$ | H | $-CH_3$ | $-CH_2[(3,4\text{-}OCH_3)C_6H_3]$ | H | H |
| $CH_3$ | H | H | $CH_3$ | H | $-CH_3$ | $-CH_2-CH_2[(3,4\text{-}OCH_3)C_6H_3]$ | H | H |
| $CH_3$ | H | H | $CH_3$ | H | $-CH_3$ | $-CH_2-[(3,4,5\text{-}OCH_3)C_6H_2]$ | H | H |
| $CH_3$ | H | H | $CH_3$ | $-COC_2H_5$ | $-CH_2-CH_2-O-CH_2-CH_2-$ | | H | H |
| $CH_3$ | H | H | $C_2H_5$ | H | $-CH_2-CH_2-N[(3,4\text{-}OCH_2O)C_6H_3]-CH_2-CH_2-$ | | H | H |

| R | $R_1$ | $R_2$ | $R_3$ | $R_4$ | $R_5$ $R_6$ | $R_7$ | $R_8$ |
|---|---|---|---|---|---|---|---|
| $CH_3$ | H | H | $CH_3$ | $C_2H_5$ | $-CH_2-CH_2-O-CH_2-CH_2-$ | $-CH=CH-CH=CH-$ | |
| $CH_3$ | H | H | $CH_3$ | H | $-CH_2-CH_2-CH_2-CH_2-$ | $-CH=CH-CH=CH-$ | |
| $CH_3$ | $CH_3$ | H | $CH_3$ | H | $-CH_2-CH_2-CH_2-CH_2-CH_2-$ | $-CH=CH-CH=CH-$ | |
| $CH_3$ | H | H | $CH_3$ | H | $-CH_2-CH_2-NH-CH_2-CH_2-$ | $-CH=CH-CH=CH-$ | |
| $CH_3$ | H | H | $CH_3$ | H | $-CH_2-CH_2-NCH_3-CH_2-CH_2-$ | $-CH=CH-CH=CH-$ | |
| $CH_3$ | H | H | $CH_3$ | H | $-CH_2-CH_2-N(CH_2-C_6H_5)-CH_2-CH_2-$ | H | H |
| $CH_3$ | H | H | $CH_3$ | H | $-CH_2-CH_2-N[CH_2-CH_2(3,4\text{-}OCH_3)C_6H_3]CH_2-CH_2$ | H | H |
| $CH_3$ | H | H | $CH_3$ | $COCF_3$ | $-CH_2-CH_2-O-CH_2-CH_2-$ | H | H |
| $CH_3$ | H | H | $CH_3$ | $-CH_2-CH_2-N(C_2H_5)_2$ | $-CH_2-CH_2-O-CH_2-CH_2-$ | H | H |

## Claims

1. A pyrrolyl-pyridazineamine derivative of the formula

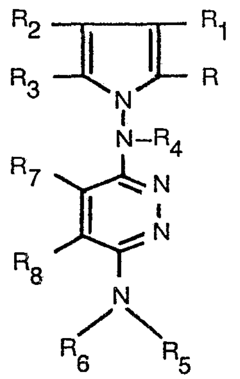

(I)

wherein R, $R_1$, $R_2$, $R_3$, may be the same or different and are independently selected from hydrogen and $(C_1—C_4)$alkyl; $R_4$ represents hydrogen, $(C_1—C_4)$alkyl, $(C_1—C_4)$alkylamino-$(C_1—C_4)$alkyl, di-$(C_1—C_4)$alkylamino$(C_1—C_4)$alkyl, $(C_1—C_4)$-alkanoyl, halo-$(C_2—C_4)$alkanoyl, carbo $(C_1—C_4)$alkoxy or carbobenzyloxy; $R_5$ and $R_6$ each independently represent $(C_1—C_4)$alkyl, hydroxy-$(C_1—C_4)$alkyl, $(C_1—C_4)$alkoxy-$(C_1—C_4)$-alkyl, $(C_1—C_4)$alkanoyloxy-$(C_1—C_4)$alkyl, $(C_3—C_4)$alkenyl, phenyl, phenyl substituted with 1 to 3 substituents independently selected from chloro, fluoro, bromo, $(C_1—C_4)$-alkyl, hydroxy, $(C_1—C_4)$alkoxy, and methylenedioxy, phenyl-$(C_1—C_4)$alkyl and substituted phenyl-$(C_1—C_4)$alkyl wherein the substituents are as above or taken together with the adjacent nitrogen atom represent a saturated 5—6 membered hererocyclic ring which may contain a further heteroatom selected from O, N and S, and which may bear 1 or 2 substituents selected from $(C_1—C_4)$alkyl, phenyl, substituted phenyl wherein the substituents are as above, phenyl-$(C_1—C_4)$alkyl, substituted phenyl-$(C_1—C_4)$alkyl wherein the substituents are as above, hydroxy, hydroxy-$(C_1—C_4)$alkyl and $(C_1—C_4)$alkanoyloxy; $R_7$ and $R_8$ represent hydrogen atoms or, taken together, a 1,3-butadienylene radical forming a benzo system fused with the pyridazine ring; and its pharmaceutically acceptable acid addition salts.

2. A compound of claim 1 wherein $R_1$ and $R_2$ are both hydrogen, R and $R_3$ are independently selected from hydrogen, methyl and ethyl; $R_4$ represents hydrogen, methyl or acetyl; $R_5$ and $R_6$ each independently represent $(C_1—C_4)$alkyl, $(C_3—C_4)$alkenyl, hydroxy-$(C_1—C_4)$alkyl, $(C_1—C_4)$alkoxy-$(C_1—C_4)$alkyl or taken together with the adjacent nitrogen atom represent a pyrrolidine, piperidine, morpholine, thiomorpholine and piperazine ring which may be optionally substituted with $(C_1—C_4)$alkyl or hydroxy on the carbon atom moiety of said rings and with $(C_1—C_4)$alkyl, phenyl and methoxyphenyl on the second nitrogen atom of the piperazine ring; $R_7$ and $R_8$ represent both hydrogen or, taken together, 1,3-butadienylene; and its pharmaceutically acceptable acid addition salts.

3. A compound of claim 1 wherein $R_1$ and $R_2$ are both hydrogen; R and $R_3$ are selected from hydrogen, methyl and ethyl, $R_4$ represents hydrogen, methyl or acetyl; $R_5$ and $R_6$ represent methyl, ethyl, allyl, 2-hydroxyethyl, 2-hydroxypropyl, 2-methoxyethyl, 2-ethoxyethyl or, taken together with the adjacent nitrogen atom represent pyrrolidine, piperidine, 4-hydroxypiperidine, morpholine, 2,6-dimethylmorpholine, thiomorpholine, piperazine, 4-methylpiperazine, 4-(2-methoxyphenyl)piperazine; $R_7$ and $R_8$ represent both hydrogen; and its pharmaceutically acceptable salts.

4. A compound of claim 1 which is N-(2,5-dimethyl-1H-pyrol-1-yl)-6-morpholino-3-pyridazineamine and its hydrochloride.

5. A compound of claim 1 which is 6-diethylamino-N-(2,5-dimethyl-1H-pyrrol-1-yl)-3-pyridazineamine.

6. A compound of claim 1 which is N-(2,5-dimethyl-1H-pyrrol-1-yl)-6-[N',N'-bis(2-methoxyethyl)amino]-3-pyridazineamine.

7. A compound of claim 1 which is N-(2,5-dimethyl-1H-pyrrol-1-yl)-6-[N',N'-bis(2-ethoxyethyl)amino]-3-pyridazineamine.

8. A process for preparing a pyrrolyl-pyridazineamine of claim 1

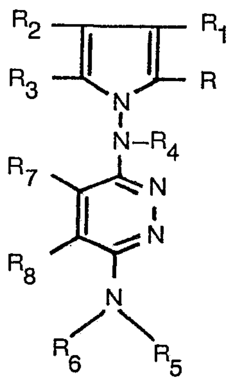

(I)

wherein R, $R_1$, $R_2$, $R_3$, $R_4$, $R_5$, $R_6$, $R_7$ and $R_8$ have the same meanings as in claim 1 which comprises contacting a hydrazino derivative of the formula

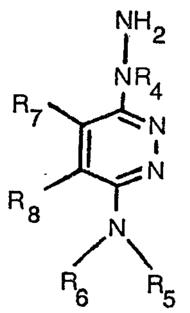

(II)

wherein $R_4$, $R_5$, $R_6$, $R_7$ and $R_8$ have the same meanings as in claim 1 or an acid addition salt thereof with a dicarbonyl compound of the formula

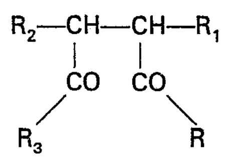

wherein R, $R_1$, $R_2$ and $R_3$ have the same meanings as in claim 1 or a functional derivative thereof wherein the keto functions can be easily restored under the reaction conditions and including the additional optional step of transforming the compounds of formula I wherein $R_4$ is hydrogen into the corresponding compound wherein $R_4$ is $(C_1—C_4)$alkyl, $(C_1—C_4)$alkanoyl, carbo$(C_1—C_4)$alkoxy, or carbobenzyloxy through common N-alkylation or N-acylation methods.

9. A process as in claim 8 wherein the reaction is carried out in the presence of an acid catalyst.

10. A process as in claim 8 wherein the reaction is carried out at a temperature between 15°C and 120°C.

11. A process as in claims 8, 9 and 10 to prepare a compound of the formula I wherein $R_1$ and $R_2$ are both hydrogen; R and $R_3$ are selected from hydrogen, methyl and ethyl; $R_4$ represents hydrogen, methyl or acetyl; $R_5$ and $R_6$ represent methyl, ethyl, allyl, 2-hydroxyethyl, 2-hydroxypropyl, 2-methoxyethyl, 2-ethoxyethyl or taken together with the adjacent nitrogen atom represent pyrrolidine, piperidine, 4-hydroxypiperidine, morpholine, 2,6-dimethylmorpholine, thiomorpholine, piperazine, 4-methylpiperazine, 4-(2-methoxyphenyl) piperazine; $R_7$ and $R_8$ represent both hydrogen and its pharmaceutically acceptable salts.

12. A process as in claim 8 for preparing a compound of formula I wherein R, $R_1$, $R_2$, $R_3$, $R_4$, $R_5$, $R_6$, $R_7$ and $R_8$ have the same meanings as in claim 1 and $R_4$ is $(C_1—C_4)$alkyl, $(C_1—C_4)$alkanoyl, carbo$(C_1—C_4)$alkoxy or carbobenzyloxy whereby the corresponding compound having $R_4$ equal to hydrogen is alkylated or acylated through a common alkylation or acylation procedure.

13. A compound of claim 1 for use in the treatment of the hypertension.

14. A pharmaceutically antihypertensive composition containing a compound of claim 1 as the active ingredient.

15. A compound of claim 13 selected from N-(2,5-dimethyl-1H-pyrrol-1-yl)-6-morpholino-3-pyridazineamine, 6-diethylamino-N-(2,5-dimethyl-1H-pyrrol-1-yl)-3-pyridazineamine, N-(2,5-dimethyl-1H-pyrrol-1-yl)-6-[N',N'-bis(2-methoxyethyl)amino]-3-pyridazineamine, N-(2,5-dimethyl-1H-pyrrol-1-yl)-6-[N',N'-bis(2-ethoxyethyl)amino]-3-pyridazineamine and their pharmaceutically acceptable acid addition salts.

16. A composition of claim 14 wherein the active ingredient is selected from N-(2,5-dimethyl-1H-pyrrol-1-yl)-6-morpholino-3-pyridazineamine, 6-diethylamino-N-(2,5-dimethyl-1H-pyrrol-1-yl)-3-pyridazineamine, N-(2,5-dimethyl-1H-pyrol-1-yl-6-[N',N'-bis(2-methoxyethyl)amino]-3-pyridazineamine, N-(2,5-dimethyl-1H-pyrrol-1-yl)-6-[N',N'-bis(2-ethoxyethyl)amino]-3-pyridazineamine and their pharmaceutically acceptable acid addition salts.

## Patentansprüche

1. Ein Pyrrolyl-pyridazinaminderivat der Formel

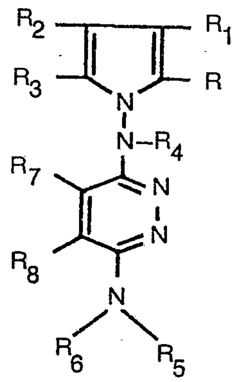

(I)

worin R, $R_1$, $R_2$ und $R_3$ gleich oder verschieden sein können und unabhängig voneinander ausgewählt sind aus Wasserstoff und ($C_1$—$C_4$)Alkyl; $R_4$ Wasserstoff, ($C_1$—$C_4$)Alkyl, ($C_1$—$C_4$)Alkylamino($C_1$—$C_4$)alkyl, Di-($C_1$—$C_4$)alkylamino($C_1$—$C_4$)alkyl, ($C_1$—$C_4$)Alkanoyl, Halogen($C_2$—$C_4$)alkanoyl, Carbo($C_1$—$C_4$)alkoxy oder Carbobenzyloxy darstellt; $R_5$ und $R_6$ unabhängig voneinander ($C_1$—$C_4$)Alkyl, Hydroxy($C_1$—$C_4$)alkyl, ($C_1$—$C_4$)Alkoxy($C_1$—$C_4$)alkyl, ($C_1$—$C_4$)Alkanoyloxy($C_1$—$C_4$)alkyl, ($C_3$—$C_4$)Alkenyl, Phenyl, Phenyl, das durch 1 bis 3 Substituenten substituiert ist, welche unabhängig voneinander ausgewählt sind aus Chlor, Fluor, Brom, ($C_1$—$C_4$)Alkyl, Hydroxy, ($C_1$—$C_4$)Alkoxy und Methylendioxy, Phenyl($C_1$—$C_4$)alkyl oder substituiertes Phenyl($C_1$—$C_4$)alkyl, worin die Substituenten wie oben angegeben sind, darstellen, oder zusammen mit dem benachbarten Stickstoffatom einen gesättigten 5- bis 6-gliedrigen heterocyclischen Ring bedeuten, der ein weiteres, aus O, N und S gewähltes Heteroatom enthalten kann, und der 1 oder 2 Substituenten, ausgewählt aus ($C_1$—$C_4$)Alkyl, Phenyl, substituiertes Phenyl, worin die Substituenten wie oben sind, Phenyl($C_1$—$C_4$)alkyl, substituiertes Phenyl($C_1$—$C_4$)alkyl, worin die Substituenten wie oben sind, Hydroxy, Hydroxy($C_1$—$C_4$)alkyl und ($C_1$—$C_4$)Alkanoyloxy tragen kann; $R_7$ und $R_8$ Wasserstoffatome oder zusammen genommen einen 1,3-Butadienylenrest bedeuten, der ein mit dem Pyridazinring kondensiertes Benzosystem bildet; und ihre pharmazeutisch annehmbaren Säureadditionssalze.

2. Eine Verbindung nach Anspruch 1, worin sowohl $R_1$ als auch $R_2$ Wasserstoff bedeuten, R und $R_3$ unabhängig voneinander ausgewählt sind aus Wasserstoff, Methyl und Ethyl; $R_4$ Wasserstoff, Methyl oder Acetyl darstellt; $R_5$ und $R_6$ unabhängig voneinander ($C_1$—$C_4$)Alkyl, ($C_3$—$C_4$)Alkenyl, Hydroxy($C_1$—$C_4$)alkyl, ($C_1$—$C_4$)Alkoxy($C_1$—$C_4$)alkyl darstellen oder gemeinsam mit dem benachbarten Stickstoffatom einen Pyrrolidin-, Piperidin-, Morpholin-, Thiomorpholin- oder Piperazinring darstellen, der gegebenenfalls am Kohlenstoffatomteil dieses Ringes durch ($C_1$—$C_4$)Alkyl oder Hydroxy und am zweiten Stickstoffatom des Piperazinrings durch ($C_1$—$C_4$)Alkyl, Phenyl und Methoxyphenyl substituiert sein kann; $R_7$ und $R_8$ jeweils Wasserstoff oder zusammen genommen 1,3-Butadienylen darstellen; und ihre pharmazeutisch annehmbaren Säureadditionssalze.

3. Eine Verbindung nach Anspruch 1, worin $R_1$ und $R_2$ jeweils Wasserstoff bedeuten; R und $R_3$ ausgewählt sind aus Wasserstoff, Methyl und Ethyl; $R_4$ Wasserstoff, Methyl oder Acetyl darstellt; $R_5$ und $R_6$ Methyl, Ethyl, Allyl, 2-Hydroxyethyl, 2-Hydroxypropyl, 2-Methoxyethyl, 2-Ethoxyethyl darstellen oder zusammen mit dem benachbarten Stickstoffatom Pyrrolidin, Piperidin, 4-Hydroxypiperidin, Morpholin, 2,6-Dimethylmorpholin, Thiomorpholin, Piperazin, 4-Methylpiperazin, 4-(2-Methoxyphenyl)piperazin darstellen; $R_7$ und $R_8$ jeweils Wasserstoff darstellen; und ihre pharmazeutisch annehmbaren Salze.

4. Eine Verbindung nach Anspruch 1, welche N-(2,5-Dimethyl-1H-pyrrol-1-yl)-6-morpholino-3-pyridazinamin ist und dessen Hydrochlorid.

5. Eine Verbindung nach Anspruch 1, welche 6-Diethylamino-N-(2,5-dimethyl-1H-pyrrol-1-yl)-3-pyridazinamin ist.

6. Eine Verbindung nach Anspruch 1, welche N-(2,5-Dimethyl-1H-pyrrol-1-yl)-6-[N',N'-bis(2-methoxyethyl)amino]-3-pyridazinamin ist.

7. Eine Verbindung nach Anspruch 1, welche N-(2,5-Dimethy-1H-pyrrol-1-yl)-6-[N',N'-bis(2-ethoxyethyl)amino]-3-pyridazinamin ist.

8. Eine Verfahren zur Herstellung eines Pyrrolyl-pyridazinamins nach Anspruch 1

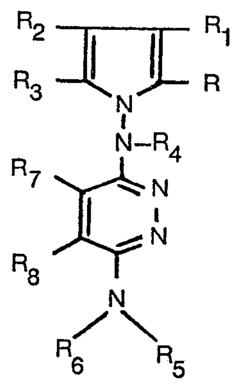

(I)

worin R, $R_1$, $R_2$, $R_3$, $R_4$, $R_5$, $R_6$, $R_7$ und $R_8$ die gleichen Bedeutungen wie in Anspruch 1 haben, umfassend das Inberührungbringen eines Hydrazinoderivats der Formel

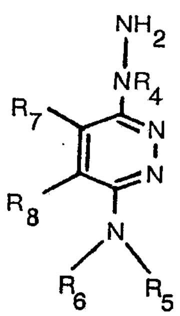

(II)

worin $R_4$, $R_5$, $R_6$, $R_7$ und $R_8$ die gleichen Bedeutungen wie in Anspruch 1 haben, oder eines Säureadditionssalzes davon mit einer Dicarbonylverbindung der Formel

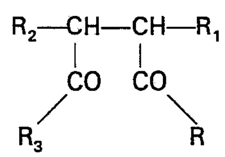

worin R, $R_1$, $R_2$ und $R_3$ die gleichen Bedeutungen wie in Anspruch 1 haben, oder einem funktionellen Derivat davon, worin die Ketofunktionen unter den Reaktionsbedingungen leicht wiederhergestellt werden können, und umfassend den zusätzlichen fakultativen Schritt des Umwandelns der Verbindungen der Formel I, worin $R_4$ Wasserstoff ist, in die entsprechende Verbindung, worin $R_4$ ($C_1$—$C_4$)Alkyl, ($C_1$—$C_4$)Alkanoyl, Carbo($C_1$—$C_4$)alkoxy oder Carbobenzyloxy ist, mittels üblicher N-Alkylierungs- oder N-Acylierungsmethoden.

9. Eine Verfahren nach Anspruch 8, worin die Reaktion in Gegenwart eines sauren Katalysators ausgeführt wird.

10. Ein Verfahren nach Anspruch 8, worin die Reaktion bei einer Temperatur zwischen 15°C und 120°C ausgeführt wird.

11. Ein Verfahren nach Anspruch 8, 9 und 10 zur Herstellung einer Verbindung der Formel I, worin $R_1$ und $R_2$ jeweils Wasserstoff sind; R und $R_3$ ausgewählt sind aus Wasserstoff, Methyl und Ethyl; $R_4$ Wasserstoff, Methyl oder Acetyl darstellt; $R_5$ und $R_6$ Methyl, Ethyl, Allyl, 2-Hydroxyethyl, 2-Hydroxypropyl, 2-Methoxyethyl, 2-Ethoxyethyl darstellen, oder zusammen mit dem benachbarten Stickstoffatom Pyrrolidin, Piperidin, 4-Hydroxypiperidin, Morpholin, 2,6-Dimethylmorpholin, Thiomorpholin, Piperazin, 4-Methylpiperazin, 4-(2-Methoxyphenyl)piperazin darstellen; $R_7$ und $R_8$ jeweils Wasserstoff darstellen, und ihrer pharmazeutisch annehmbaren Salze.

12. Ein Verfahren nach Anspruch 8 zur Herstellung einer Verbindung der Formel I, worin R, $R_1$, $R_2$, $R_3$, $R_4$, $R_5$, $R_6$, $R_7$ und $R_8$ die gleiche Bedeutung haben wie in Anspruch 1 und $R_4$ ($C_1$—$C_4$)Alkyl, ($C_1$—$C_4$)Alkanoyl, Carbo($C_1$—$C_4$)alkoxy oder Carbobenzyloxy ist, wobei die entsprechende Verbindung, die $R_4$ gleich Wasserstoff hat, mittels üblicher Alkylierungsoder Acylierungsverfahren alkyliert oder acyliert wird.

13. Eine Verbindung nach Anspruch 1 zur Verwendung bei der Behandlung der Hypertonie.

14. Eine pharmazeutische Zusammensetzung gegen Hypertonie, enthaltend eine Verbindung nach Anspruch 1 als wirksamen Bestandteil.

15. Eine Verbindung nach Anspruch 13, ausgewählt aus N-(2,5-Dimethyl-1H-pyrrol-1-yl)-6-morpholino-3-pyridazinamin, 6-Diethylamino-N-(2,5-dimethyl-1H-pyrrol-1-yl)-3-pyridazinamin, N-(2,5-Dimethyl-1H-pyrrol-1-yl)-6-[N',N'-bis(2-methoxyethyl)amino]-3-pyridazinamin, N-(2,5-Dimethyl-1H-pyrrol-1-yl)-6-[N',N'-bis(2-ethoxyethyl)amino]-3-pyridazinamin, und ihre pharmazeutisch annehmbaren Säureadditionssalze.

16. Eine Zusammensetzung nach Anspruch 14, worin der Aktivbestandteil ausgewählt ist aus N-(2,5-Dimethyl-1H-pyrrol-1-yl)-6-morpholino-3-pyridazinamin, 6-Diethylamino-N-(2,5-dimethyl-1H-pyrrol-1-yl)-3-pyridazinamin, N-(2,5-Dimethyl-1H-pyrrol-1-yl)-6-[N',N'-bis(2-methoxyethyl)amino]-3-pyridazinamin, N-(2,5-Dimethyl-1H-pyrrol-1-yl)-6-[N',N'-bis(2-ethoxyethyl)amino]-3-pyridazinamin und ihren pharmazeutisch annehmbaren Säureadditionssalzen.

## Revendications

1. Dérivé de pyrrolyl-pyridazineamine possédant la formule suivante:

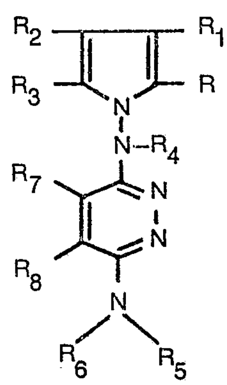

(I)

dans laquelle R, $R_1$, $R_2$, $R_3$ peuvent être identiques ou différents et sont indépendamment choisis parmi hydrogène et alcoyle en $C_1$ à $C_4$; $R_4$ représente hydrogène, alcoyle en $C_1$ à $C_4$, alcoyl($C_1$—$C_4$)aminoalcoyle en $C_1$ à $C_4$, dialcoyl($C_1$—$C_4$)aminoalcoyle en $C_1$ à $C_4$, alcanoyle en $C_1$ à $C_4$, halogéno-alcanoyle en $C_2$ à $C_4$, carbo-alcoxy en $C_1$ à $C_4$ ou carbobenzyloxy; $R_5$ et $R_6$ représentent chacun indépendamment alcoyle en $C_1$ à $C_4$, hydroxyalcoyle en $C_1$ à $C_4$, alcoxy($C_1$—$C_4$)alcoyle en $C_1$ à $C_4$, alcanoyloxy($C_1$—$C_4$) alcoyle en $C_1$ à $C_4$, alcényle en $C_3$ à $C_4$, phényle, phényle substitué avec de 1 à 3 substituants indépendamment choisis parmi chloro, fluoro, bromo, alcoyle en $C_1$ à $C_4$, hydroxy, alcoxy en $C_1$ à $C_4$ et méthylènedioxy, phényl-alcoyle en $C_1$ à $C_4$ et phényle(substitué)alcoyle en $C_1$ à $C_4$ dans lequel les substituants sont tels que spécifiés ci-dessus ou bien, considérés conjointement avec l'atome d'azote adjacent, représentent un hétérocycle saturé à 5 ou 6 maillons qui peut contenir un autre hétéroatome choisi parmi O, N et S et qui peut porter 1 ou 2 substituants choisis parmi alcoyle en $C_1$ à $C_4$, phényle, phényle substitué dans lequel les substituants sont comme ci-dessus, phénylalcoyle en $C_1$ à $C_4$, phényl(substitué)alcoyle en $C_1$ à $C_4$ dans lequel les substituants sont comme ci-dessus, hydroxy, hydroxyalcoyle en $C_1$ à $C_4$ et alcanoyloxy en $C_1$ à $C_4$; $R_7$ et $R_8$ représentent des atomes d'hydrogène ou bien, considérés conjointement, un radical 1,3-butadiénylène formant un système benzo fusionné avec le cycle pyridazine; et les sels d'addition d'un tel dérivé avec des acides pharmaceutiquement acceptables.

2. Composé selon la revendication 1 dans lequel $R_1$ et $R_2$ sont tous deux hydrogène; R et $R_3$ sont indépendamment choisis parmi hydrogène, méthyle et éthyle; $R_4$ représente hydrogène, méthyle ou acétyle; $R_5$ et $R_6$ représentent chacun indépendamment alcoyle en $C_1$ à $C_4$, alcényle en $C_3$ à $C_4$, hydroxyalcoyle en $C_1$ à $C_4$, alcoxy($C_1$—$C_4$)alcoyle en $C_1$ à $C_4$ ou bien, considérés conjointement avec l'atome d'azote adjacent, représentent un cycle pyrrolidine, pipéridine, morpholine, thiomorpholine et pipérazine qui peut être facultativement substitué avec alcoyle en $C_1$ à $C_4$ ou hydroxy sur la portion atome de carbone desdits cycles et avec alcoyle en $C_1$ à $C_4$, phényle et méthoxyphényle sur le second atome d'azote du cycle pipérazine; $R_7$ et $R_8$ représentent tous deux de l'hydrogène ou bien, considérés conjointement, 1,3-butadiénylène; et ses sels avec des acides pharmaceutiquement acceptables (sels d'addition).

3. Composé selon la revendication 1 dans lequel $R_1$ et $R_2$ sont tous deux hydrogène; R et $R_3$ sont choisis parmi hydrogène, méthyle et éthyle; $R_4$ représente hydrogène, méthyle ou acétyle; $R_5$ et $R_6$ représentent méthyle, éthyle, allyle, 2-hydroxyéthyle, 2-hydroxypropyle, 2-méthoxyéthyle, 2-éthoxyéthyle ou bien, considérés conjointement avec l'atome d'azote adjacent, représentent pyrrolidine, pipéridine, 4-hydroxypipéridine, morpholine, 2,6-diméthyl-morpholine, thiomorpholine, pipérazine, 4-méthylpipérazine, 4-(2-méthoxyphényl)-pipérazine; $R_7$ et $R_8$ représentent tous deux de l'hydrogène; et ses sels pharmaceutiquement acceptables.

4. Composé selon la revendication 1 qui est de la N-(2,5-diméthyl-1H-pyrrol-1-yl)-6-morpholino-3-pyridazineamine et son chlorhydrate.

5. Composé selon la revendication 1 qui est de la 6-diéthylamino-N-(2,5-diméthyl-1H-pyrrol-1-yl)-3-pyridazineamine.

6. Composé selon la revendication 1 qui est de la N-(2,5-diméthyl-1H-pyrrol-1-yl)-6-[N',N'-bis(2-méthoxyéthyl)amino]-3-pyridazineamine.

7. Composé selon la revendication 1 qui est de la N-(2,5-diméthyl-1H-pyrrol-1-yl)-6-[N',N'-bis(2-éthoxyéthyl)amino]-3-pyridazineamine.

8. Procédé pour préparer une pyrrolyl-pyridazineamine selon la revendication 1

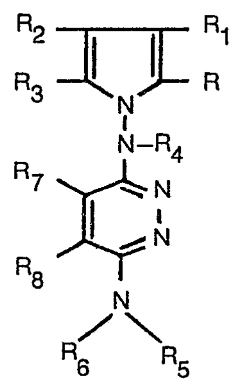

(I)

dans laquelle R, $R_1$, $R_2$, $R_3$, $R_4$, $R_5$, $R_6$, $R_7$ et $R_8$ ont les mêmes significations que dans la revendication 1, qui comprend la mise en contact d'un dérivé hydrazino possédant la formule suivante:

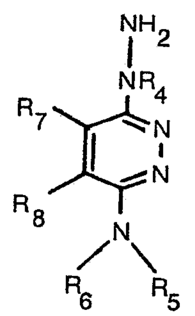

(II)

dans laquelle $R_4$, $R_5$, $R_6$, $R_7$ et $R_8$ ont les mêmes significations que dans la revendication 1, ou un sel d'addition d'une telle substance avec un acide, avec un composé diacarbonylé possédant la formule suivante:

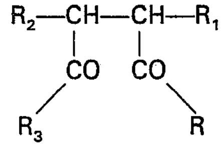

dans laquelle R, $R_1$, $R_2$ et $R_3$ ont les mêmes significations que dans la revendication 1, ou un dérivé fonctionnel d'un tel composé dicarbonylé dans lequel les fonctions céto peuvent être facilement reconstituées dans les conditions de réaction, et qui comprend en outre l'opération élémentaire facultative additionelle consistant à transformer les composés possédant la formule (I) dans laquelle $R_4$ est de l'hydrogène en le composé correspondant dans lequel $R_4$ est alcoyle en $C_1$ à $C_4$, alcanoyle en $C_1$ à

$C_4$, carbo-alcoxy en $C_1$ à $C_4$, ou carbobenzyloxy, par mise en oeuvre de méthodes classiques de N-alcoylation ou de N-acylation.

9. Procédé selon la revendication 8, dans lequel on effectue la réaction en présence d'un catalyseur acide.

10. Procédé selon la revendication 8, dans lequel on effectue la réaction à une température comprise entre 15°C et 120°C.

11. Procédé selon les revendications 8, 9 et 10 pour préparer un composé possédant la formule (I) dans laquelle $R_1$ et $R_2$ sont tous deux hydrogène; R et $R_3$ sont choisis parmi hydrogène, méthyl et éthyle; $R_4$ représente hydrogène, méthyle ou acétyle; $R_5$ et $R_6$ représentent méthyle, éthyle, allyle, 2-hydroxyéthyle, 2-hydroxypropyle, 2-méthoxyéthyle, 2-éthoxyéthyle ou bien, considérés conjointement avec l'atome d'azote adjacent, représentent pyrrolidine, pipéridine, 4-hydroxypipéridine, morpholine, 2,6-diméthylmorpholine, thiomorpholine, pipérazine, 4-méthylpipérazine, 4-(2-méthoxyphényl)pipérazine; $R_7$ et $R_8$ représentent tous deux de l'hydrogène; et les sels pharmaceutiquement acceptables d'un tel composé.

12. Procédé selon la revendication 8 pour préparer un composé possedant la formule I dans laquelle R, $R_1$, $R_2$, $R_3$, $R_4$, $R_5$, $R_6$, $R_7$ et $R_8$ ont les mêmes significations que dans la revendication 1 et $R_4$ est alcoyle en $C_1$ à $C_4$, alcanoyle en $C_1$ à $C_4$, carboalcoxy en $C_1$ à $C_4$ ou carbobenzyloxy; procédé au cours; duquel le composé correspondant possédant un $R_4$ qui est de l'hydrogène est alcoylé ou acylé par mise en oeuvre d'une méthode classique d'alcoylation ou d'acylation.

13. Composé selon la revendication 1 utilisable pour le traitement de l'hypertension.

14. Composition pharmaceutiquement antihypertensive contenant un composé selon la revendication 1 comme ingrédient actif.

15. Composé selon la revendication 13 choisi parmi: N-(2,5-diméthyl-1H-pyrrol-1-yl)-6-morpholino-3-pyridazineamine; 6-diéthylamino-N-(2,5-diméthyl-1H-pyrrol-1-yl)-3-pyridazineamine; N-(2,5-diméthyl-1H-pyrrol-1-yl-6-[N',N'-bis(2-méthoxyéthyl)amino]-3-pyridazineamine; N-(2,5-diméthyl-1H-pyrrol-1-yl)-6-[N',N'-bis(2-éthoxyéthyl)amino]-3-pyridazineamine, et leurs sels d'addition avec des acides pharmaceutiquement acceptables.

16. Composition selon la revendication 14 dans laquelle l'ingredient actif est choisi parmi: N-(2,5-diméthyl-1H-pyrrol-1-yl)-6-morpholino-3-pyridazineamine; 6-diéthylamino-N-(2,5-diméthyl-1H-pyrrol-1-yl)-3-pyridazineamine; N-(2,5-diméthyl-1H-pyrrol-1-yl)-6-[N',N'-bis(2-méthoxyéthyl)amino]-3-pyradizineamine; N-(2,5-diméthyl-1H-pyrrol-1-yl)-6-[N',N'-bis(2-éthoxyéthyl)amino]-3-pyradizineamine, et leurs sels d'addition avec des acides pharmaceutiquement acceptables.